(19) Europäisches Patentamt
European Patent Office
Office européen des brevets

(11) Publication number : 0 481 708 A1

# (12) EUROPEAN PATENT APPLICATION

(21) Application number : 91309445.4

(22) Date of filing : 15.10.91

(51) Int. Cl.[5] : C07D 209/94, A61K 31/40

(30) Priority : 16.10.90 GB 9022453
21.12.90 GB 9027895

(43) Date of publication of application :
22.04.92 Bulletin 92/17

(84) Designated Contracting States :
AT BE CH DE DK ES FR GB GR IT LI LU NL SE

(71) Applicant : UNIVERSITY OF BATH
School of Chemistry
Claverton Down, Bath, BA2 7AY (GB)

(71) Applicant : UNIVERSITY OF CINCINNATI
Administration Building
Cincinnati, Ohio 45221-0627 (US)

(72) Inventor : Sainsbury, Malcolm
3 Homefield Close
Saltford, Bristol BS18 3EF (GB)
Inventor : Shertzer, Howard G.
1819 Fireside Drive
Cincinnati, Ohio 45265 (US)

(74) Representative : Marshall, Monica Anne et al
J.A. Kemp & Co. 14 South Square Gray's Inn
London WC1R 5LX (GB)

## (54) Indenoindole compounds.

(57) Cis-fused compounds of the formula I

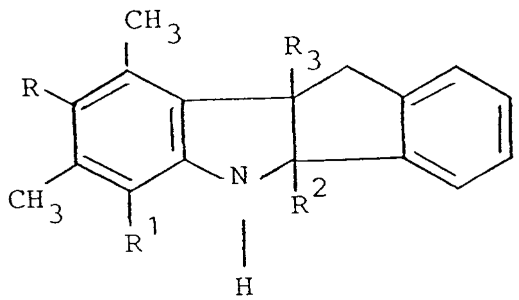

I

wherein R is methoxy or ethoxy, when R is methoxy, $R^1$ is methyl and $R^2$ and $R^3$ are both hydrogen or both methyl, and when R is ethoxy, $R^1$ is hydrogen and $R^2$ and $R^3$ are both methyl, and enantiomers and salts thereof are new and useful as antioxidants, within the medical and non-medical field.

EP 0 481 708 A1

## Field of the Invention

The present invention relates to novel indenoindole compounds, which are hydrophobic antioxidants, and highly efficient in reducing, i.e. quenching, free radicals in lipids or lipid biphases, thereby terminating the lipid peroxidation process and preventing conditions and diseases initiated by this or related processes. The invention also relates to compositions, especially pharmaceutical compositions, containing at least one compound of the invention, or a salt thereof, especially a therapeutically acceptable salt thereof, as active ingredient. In further aspects, the invention relates to processes for the preparation of such compounds and to the use of the active compounds in medical therapy and prevention as well as in non-medical applications. Especially important in non-medical applications would be the use in controlling or terminating free-radical mediated processes.

## Background of the Invention

Some biological processes generate more or less stable intermediates that contain an unpaired electron, which can either be donated, or paired with an additional electron from the surroundings. Such intermediates are called free radicals, and they may be the products of various enzymatic and non-enzymatic reactions, some of which are vital for body functions, e.g. reduction of ribonucleoside diphosphates for DNA synthesis and the generation of prostaglandins in the prostaglandin synthase reaction. The latter is essential for inflammatory response following cell injury, and a number of other functions. Other radical reactions include the myeloperoxidase reaction in neutrophils and macrophages which destroy bacteria and other invading particles, and the electron transport in the mitochondrial respiratory chain. Most organisms contain chemical antioxidants such as α-tocopherol (vitamin E), ascorbic acid and different radical and peroxide-inactivating enzymes, e.g. superoxide dismutase, catalase and glutathione peroxidase.

Free radicals of various types are becoming increasingly associated with a broad range of conditions and diseases such as ischemic or reperfusion injury, atherosclerosis, thrombosis and embolism, allergic/inflammatory conditions such as bronchial asthma, rheumatoid arthritis, conditions related to Alzheimer's disease, Parkinson's disease and ageing, cataract, diabetes, neoplasms and toxicity of anti-neoplastic or immunosuppressive agents and chemicals. One possible explanation for these conditions and diseases is that, for unknown reasons, the endogeneous protecting agents against radical damage are not sufficiently active to protect the tissue against radical damage. Lipid peroxidation caused by excess generation of radicals may constitute one significant damaging pathway in the above conditions and diseases. Administration of additional antioxidants, which inhibit radical reactions, e.g. lipid peroxidation, would thus provide a way of preventing or curing the above conditions and diseases. The present invention describes new antioxidants of the indenoindole type that fulfil both the requirement to accumulate in membranes, i.e. they are sufficiently hydrophobic, and they are potent inhibitors of lipid peroxidation. These new antioxidants compare favourably with other antioxidants, e.g. α-tocopherol. The compounds of the present invention may also be used in non-medical applications for stabilising compounds susceptible to oxidative deterioration, e.g. in skin care products, food preservation, food additives and for preservation of other products. The present invention extends to both a method of stabilisation using the tetrahydroindenoindoles and the resulting stabilised compositions.

## Prior Art

Our prior patent application PCT/GB90/00949, published as International specification WO90/15800 on 27 December 1990, relates to a group of tetrahydroindenoindoles which are effective as inhibitors of the lipid peroxidation process and useful as antioxidants.

## Disclosure of the Invention

According to the present invention, there are provided cis-fused compounds of the formula I

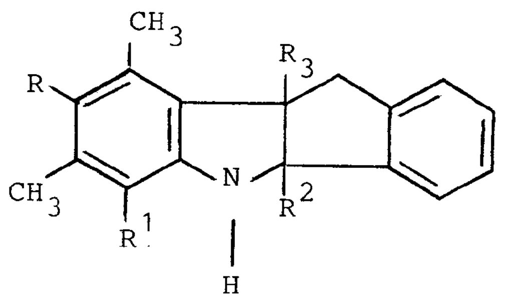

I

wherein R is methoxy or ethoxy, when R is methoxy, $R^1$ is methyl and $R^2$ and $R^3$ are both hydrogen or both methyl, and when R is ethoxy, $R^1$ is hydrogen and $R^2$ and $R^3$ are both methyl, and enantiomers and salts thereof.

It has been found that cis-fused compounds with the tetrahydroindenoindole structures of formula I are highly effective as inhibitors of the lipid peroxidation process and useful as antioxidants. The compounds of formula I may be present as a racemic mixture or as the pure enantiomers or as combinations thereof.

The compounds of the invention are particularly useful as antioxidants in the medical therapy.

The indole structures of the present invention have the following numbering in the rings.

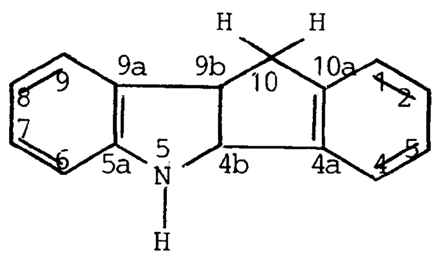

cis-4b,5,9b,10-Tetrahydroindeno[1,2-b]indole(THII)

Tetrahydroindenoindoles having the formula I, which are included in the present invention are the following:
cis-4b,5,9b,10-tetrahydro-4b,6,7,9,9b-pentamethyl-8-methoxy-indeno[1,2-b]indole
cis-4b,5,9b,10-tetrahydro-8-ethoxy-4b,7,9,9b-tetramethyl-indeno[1,2-b]indole
cis-4b,5,9b,10-tetrahydro-8-methoxy-6,7,9-trimethyl-indeno[1,2-b]indole

Pharmaceutical preparations

According to the present invention the cis-fused compounds of the formula I will normally be administered orally, rectally, dermally or by injection, in the form of pharmaceutical preparations comprising the active ingredient either as a free base or a pharmaceutically acceptable non-toxic acid addition salt, e.g. the hydrochloride, hydrobromide, lactate, acetate, phosphate, sulfate, sulfamate, citrate, tartrate, oxalate and the like in a pharmaceutically acceptable dosage form. The preparations may contain a pharmaceutically acceptable carrier or diluent.

The dosage form may be a solid, semisolid or liquid preparation. Usually the active substance will constitute between 0.1 and 99% by weight of the preparation, more specifically between 0.5 and 20% by weight for preparations intended for injection and between 0.2 and 50% by weight for preparations suitable for oral administration. Dermal administration would normally utilize 0.1 - 5% by weight of the active ingredient in a suitable vehicle.

To produce pharmaceutical preparations containing a compound of the invention in the form of dosage units for oral application, the selected compound may be mixed with a solid excipient, e.g. lactose, saccharose, sorbitol, mannitol, starches such as potato starch, corn starch or amylopectin, cellulose derivatives, a binder such as gelatine or poly-vinylpyrrolidone, and a lubricant such as magnesium stearate, calcium stearate, polyethylene glycol, waxes, paraffin, and the like, and then compressed into tablets. If coated tablets are required, the cores, prepared as described above, may be coated with a concentrated sugar solution which may contain e.g. gum arabic, gelatine, talcum, titanium dioxide, and the like. Alternatively, the tablet can be croated with a polymer known to the man skilled in the art, dissolved in a readily volatile organic solvent or mixture of organic solvents. Dyestuffs may be added to these coatings in order to readily distinguish between tablets containing different active substances or different amounts of the active compounds.

For the preparation of soft gelatine capsules, the active substance may be admixed with e.g. a vegetable oil or poly-ethylene glycol. Hard gelatine capsules may contain granules of the active substance using either the above-mentioned excipients for tablets e.g. lactose, saccharose, sorbitol, mannitol, starches (e.g. potato starch, corn starch or amylopectin), cellulose derivatives or gelatine. Also liquids or semisolids of the drug can be filled into hard gelatine capsules.

Dosage units for rectal application can be solutions or suspensions or can be prepared in the form of suppositories comprising the active substance in admixture with a neutral fatty base, or gelatine rectal capsules comprising the active substance in admixture with vegetable oil or paraffin oil.

Liquid preparations for oral application may be in the form of syrups or suspensions, for example solutions containing from about 0.2% to about 20% by weight of the active substance herein described, the balance being sugar and mixture of ethanol, water, glycerol and propylene glycol. Optionally such liquid preparations may contain colouring agents, flavouring agents, saccharine and carboxymethyl-cellulose as a thickening agent or other excipients known to the man in the art.

Solutions for parenteral applications by injection can be prepared in an aqueous solution of a water-soluble pharmaceutically acceptable salt of the active substance, preferably in a concentration of from about 0.5% to about 10% by weight. These solutions may also contain stabilizing agents and/or buffering agents and may conveniently be provided in various dosage unit ampoules.

Suitable daily doses of the compounds of the invention in therapeutical treatment of humans are about 0.01-100 mg/kg body weight at peroral administration and 0.001-100 mg/kg body weight at parenteral administration.

Method of Preparation

The compounds of the invention may be prepared as outlined below, however, the invention is not limited to these methods, the compounds may be prepared by processes described in known art.

a. The 4b,5,9b,10-tetrahydroindeno[1,2-b]indole (IA) wherein $R^2$ and $R^3$ are both hydrogen may be prepared by reduction of the corresponding 5,10-dihydroindeno[1,2-b]indole (DHII)

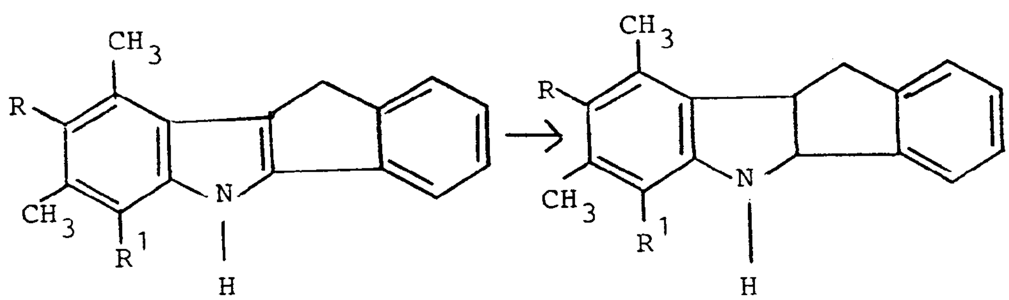

II IA

wherein R and $R^1$ are as defined under formula I.

The DHII starting material is reduced by reaction with a boron based reductant such as sodium cyanoborohydride in a solvent, often acetic acid, or $BH_3$ in tetrahydrofurane. Alternatively morpholino borane in a solvent, often tetrahydrofurane or dioxane, and in the presence of a strong acid e.g. hydrochloric acid, can be used. Alternatively a trialkylsilane can be used. At the end of the reaction the product is isolated by dilution of the reaction mixture with water, neutralisation, and either filtration or solvent extraction. Alternatively reduction is achieved by hydrogenation over a catalyst such as palladium, in this case the DHII compound is dissolved in a suitable solvent, for example ethanol, acetic acid, or ethyl acetate. In such case the product is isolated by removal of the catalyst and evaporation of the solvent under reduced pressure. The THII compound may be purified by crystallization from a suitable solvent, or by column chromatography using silica.

b. 4b,9b-Dimethyl-4b,5,9b,10-tetrahydroindeno[1,2-b]indoles (IB) may be prepared directly by reacting indolenines of formula III with methyl lithium ($CH_3Li$) in an aprotic solvent such as dry tetrahydrofuran.

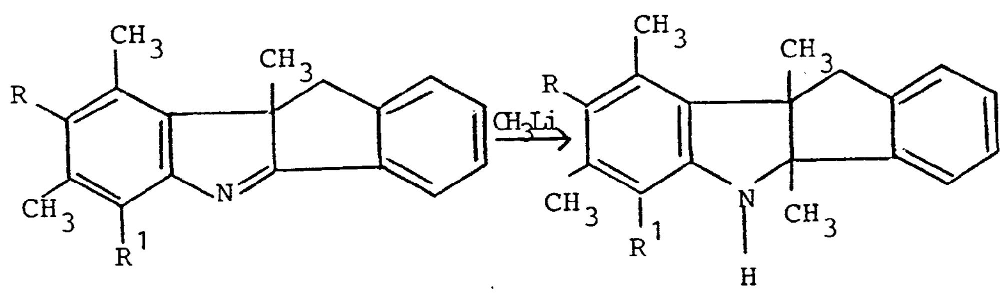


III IB

wherein R and $R^1$ are as defined under formula I.

c. 4b,9b-Dimethyl-4b,5,9b,10-tetrahydroindeno[1,2-b]indoles (IB) may alternatively be prepared from the corresponding 4b-unsubstituted analogues by a sequence of metallation, e.g. using butyl lithium, carbonation with carbon dioxide, a second directed metallation, e.g. using butyl lithium, and an alkylation with methyl-halide or methyl-sulphate followed by a final hydrolysis of the resulting N-carboxylated intermediates.

Preparation of Starting Materials

The DHII starting materials of formula II may be synthesized by the Fischer indolisation of the phenylhydrazine of formula IV with 1-indanone of formula V ($R_4$ being hydrogen).

The indolenine starting materials of formula III may be synthesized by reacting a phenylhydrazine of formula IV with 2-methyl-1-indanone of formula V ($R_4$ being methyl).

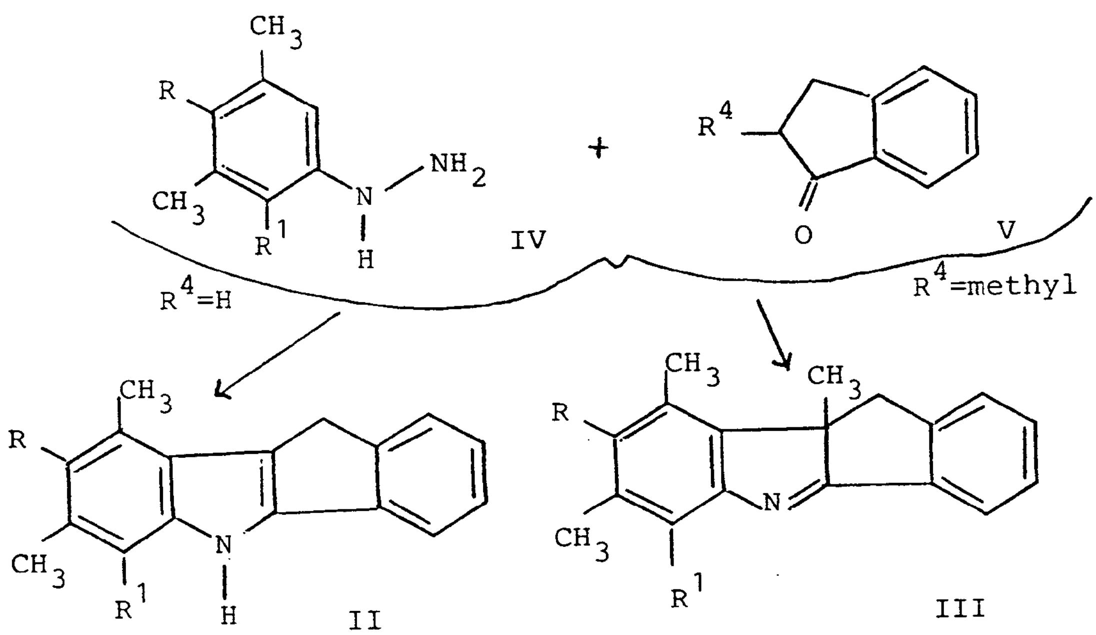


IV V

II III

wherein R and $R^1$ are as defined under formula I and $R^4$ is hydrogen or methyl, respectively.

The 1- or 2-methyl-1-indanones of formula V may be reacted with the phenylhydrazines of formula IV either as the free base, or as a salt, often the hydrochloride. Normally the reactants are dissolved in a solvent preferably an alcoholic solvent such as ethanol or propanol. In some cases heat is not required, whereas in others it is necessary to heat the reaction mixture to reflux for up to 1 hour, or more.

The reaction of the compound of formula V and the phenylhydrazines of formula IV to form a compound of formula II or III proceeds via a phenylhydrazone intermediate of formula VI

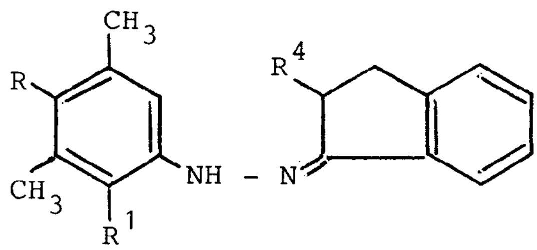

VI

where R and $R^1$ are as defined under formula I and $R^4$ is hydrogen or methyl.

The phenylhydrazone intermediate can sometimes be isolated by dilution of the reaction mixture with water and separated by filtration, or by extraction with a suitable solvent. Further purification is achieved by crystallisation or by chromatography. In the last case column chromatography on silica is satisfactory and a range of eluting solvents may be used.

Cyclisation of the phenylhydrazones to the DHII of formula II or indolenines of formula III can be achieved by redissolving them in a suitable solvent, preferably an alcohol such as ethanol or propanol, and treating the solution with an acid, for example, hydrochloric acid, acetic acid, or trifluroacetic acid. Heat may or may not be required. Other cyclisation reagents including Lewis acids such as zinc chloride, or reagents containing a phosphorus atom, for example phosphorus trichloride, phosphorus oxytrichloride, polyphosphoric acid, or polyphosphonates, can also be used.

Should the salts of phenylhydrazines be used in place of phenylhydrazines in reactions with the indanones then cyclisation of the intermediate phenylhydrazones to the DHII or indolenines may occur spontaneously.

The 4b-unsubstituted analogues of the compounds of formula IB i.e. compounds of the formula VII

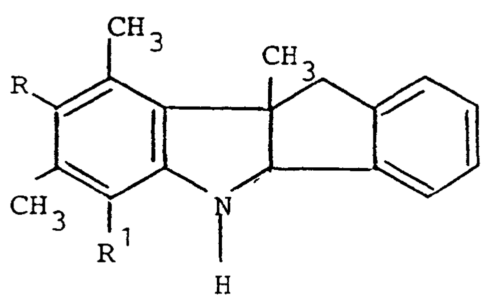

VII

can be synthesized by the Fischer indolisation of a phenylhydrazine of formula IV and the 2-methyl-1-indanone of formula V ($R^4$ being methyl) followed by the reduction of the intermediate indolenine of formula III.

Reduction of the indolenines of formula III to the THII derivatives substituted at C-9b is achieved using standard reducing agents such as sodium borohydride in an appropriate solvent such as ethanol. The products are then isolated and purified in the usual way.

Processes for preparation of starting materials such as 5,10-dihydroindeno[1,2-b]indole (DHII) and analogues containing functional groups are described in our application EP-A-0 404 536.

The following illustrates the principle and the adaption of the invention, however, without being limited thereto. Temperature is given degrees Celcius.

## Working Examples

### Example 1

cis-4b,5,9b,10-tetrahydro-4b,6,7,9,9b-pentamethyl-8-methoxyindeno[1,2-b]indole

i) 2,3,6-trimethylanisole

A mixture of 50g (0.367 mol) of 2,3,6-trimethylphenol, 55g (0.4 mol) of potassium carbonate and 36 ml (0.38 mol) of dimethylsulphate in 500 ml of acetonitrile was heated under reflux overnight. 35 ml of conc. aqueous ammonia was then added, and the mixture refluxed for 1 hour. After filtration and evaporation, the residue was taken up in ether and washed twice with NaOH-solution and twice with $NaHCO_3$-solution. Drying ($MgSO_4$) and evaporation gave 50.8g (92%) of the product.

ii) 4-nitro-2,3,6-trimethylanisole

To a stirred, heated (70-80°C) solution of 20.3g (0.135 mol) of 2,3,6-trimethylanisole in 200 ml of acetic acid was added dropwise 9.65 ml of conc. aqueous $HNO_3$ in 20 ml of acetic acid. After the addition the mixture was poured onto ice, and the resulting mixture was extracted 3 times with methylene chloride. The organic phase was evaporated to remove acetic acid and methylene chloride, and the residue was dissolved in ether and washed twice with NaOH-solution and twice with $NaHCO_3$-solution. Drying ($MgSO_4$) and evaporation gave 19.0g (72%) of the product, which was used without further purification in the next step.

iii) 4-amino-2,3,6-trimethylanisole

A mixture of 19.0g (0.097 mol) of 4-nitro-2,3,6-trimethylanisole and 2 small spoons of Pd/C (5%) in 200 of ethanol was hydrogenated for 4 hours in a Parr apparatus. The catalyst was then removed by filtration and the solvent evaporated. The intensive redish residue was dissolved in ether and extracted with aqueous HCl (2M).

The combined aqueous phases were then alkalinized with NaOH-solution to pH 14. The resulting basic solution was extracted with ether. After drying ($MgSO_4$), the product was precipitated as its HCl-salt by addition of a solution of HCl (g) in ether. Filtration gave 10.1g (63%) of the product.

iv) 4-hydrazino-2,3,6-trimethylanisole

A suspension of 5.17g (0.0256 mol) of 4-amino-2,3,6-trimethylanisole hydrochloride in 30 ml of conc. aqueous HCl and 35 ml of water was heated (80°C) until the hydrochloride dissolved. Cooling to -5°C resulted in a reprecipitation of the hydrochloride as small fine crystals. To this stirred mixture 1.77g (0.0256 mol) of $NaNO_2$ in 15 ml of water was added during 30 minutes, while the reaction temperature was kept between -5°C to 0°C. After stirring at this temperature for 15 minutes further, a solution of 14.46g (0.064 mol) of $SnCl_2x2H_2O$ in 12 ml of conc. aqueous HCl was added dropwise during 15 minutes while keeping the temperature at 0°C. The reaction mixture was then allowed slowly to attain room temperature and was then alkalinized with NaOH-solution until pH 14. The resulting mixture was extracted with ether, and after drying ($MgSO_4$) the product was precipitated as its hydrochloride by addition of HCl (g) in ether. Filtration gave 3.8 g (69%) of the product.

v) 9b,10-dihydro-6,7,9,9b-tetramethyl-8-methoxyindeno-[1,2-b]indole

A mixture of 2.2g (0.012 mol) of 4-hydrazino-2,3,6-trimethylanisole, 1.9g (0.013 mol) of 2-methyl-1-indanone and 2 ml of conc. aqueous HCl in 20 ml of ethanol was refluxed under argon for 1 hour. After cooling the solvent was removed by evaporation and the residue was partitioned in a mixture of water and methylene chloride. The aqueous phase was neutralized by addition of aqueous $NaHCO_3$ and the phases separated. After drying ($MgSO_4$) and evaporation of the organic phase, the remaining crude product was purified by chromatography on silica using methylene chloride as an eluant. This gave 1.1g 31% of the product which was used directly in the next step.

vi) cis-4b,5,9b,10-tetrahydro-4b,6,7,9,9b-pentamethyl-8-methoxyindeno[1,2b]indole

To a cold (-80°C) solution of 1.1g (0.0038 mol) of 9b,10-dihydro-6,7,9,9b-tetramethyl-8-methoxyindeno[1,2-b] indole in 20 ml of dry THF was added 4 ml of 1.6M methyl lithium in ether under argon. The mixture was stirred at -78°C for 1 hour and then at -20°C for 1 hour. After attaining 0°C aqueous $NH_4Cl$ and ether was added. The phases were separated, and the organic phase was washed once with water, dried ($MgSO_4$) and evaporated. The resulting crude product was purified by chromatography on silica using ethyl acetate/isooctane (2/10) as eluant. This gave 0.3g (25%) of the expected product. $^1H$ NMR ($CDCl_3$) :1.41 (3H,s), 1.43 (3H,s), 1.96 (3H,s), 2.07 (3H,s), 2.35 (3H,s), 3.0 (1H,d), 3.55 (3H,s), 3.65 (1H,d), 7.1 (3H,m), 7.25 (1H,m).

EXAMPLE 2

Cis-4b,5,9b,40-tetrahydro-8-ethoxy-4b,7,9,9b-tetramethylindeno[1,2-b]indole.

i) 4-nitro-2,6-dimethyl-1-ethoxybenzene

A mixture of 10 g (0.0598 mol) of 4-nitro-2,6-dimethylphenol, 16.5 g (0.1196 mol) of $K_2CO_3$ and 18.6 g (0.1196 mol) of ethyl iodide in 100 ml of acetonitrile was refluxed for 2.5 hours. The solid material was removed by hot filtration and was then washed with hot acetonitrile. The combined organic phase was evaporated and the resulting residue dissolved in ether. The ether phase was washed with water, dried ($Na_2SO_4$) and evaporated yielding 11.4 g (98%) of the product, M.p. 56°C.

ii) 3,5-dimethyl-4-ethoxyaniline

A solution of 11.4 g (0.058 mol) of 4-nitro-2,6-methyl-1-ethoxybenzene in 115 ml of ethanol (95%) was hydrogenated using Pd/C as catalyst overnight. After removal of the catalyst by filtration the solvent was removed by evaporation yielding 9.2 g (96%) of the product, M.p. 74°C.

iii) 3,5-dimethyl-4-ethoxyphenylhydrazine

To a cooled (+5°C), stirred suspension of 9.2 g (0.0557 mol) of 3,5-dimethyl-4-ethoxyaniline in 50 ml of 6N HCl (aq), 3.8 g (0.0557 mol) of $NaNO_2$ in 15 ml of water was added during 30 minutes. After stirring at +5°C for 30 minutes further, the resulting mixture was added under argon and stirring to 29.1 g (0.167 mol) of sodium dithionite dissolved in 150 ml of water.

After stirring for 20 minutes at +5°C, 250 ml of ether was added followed by alkalinization to pH 9 with 10 N sodium hydroxide solution. The organic phase was separated and washed with sodium chloride solution. After drying ($Na_2SO_4$) the product was precipitated as the hydrochloride by addition of HCl(g)/ether (to pH 3). Filtration and washing with ether gave 9.4g (78%) of the product.

iv) 9b,10-dihydro-8-ethoxy-7,9,9b-trimethylindeno[1,2-b]indole

A solution of 2.16 g (0.01 mol) of 3,5-dimethyl-4-ethoxyphenylhydrazine hydrochloride and 1.46 g (0.01 mol) of 2-methyl-1-indanone in 20 ml of acetic acid was stirred overnight at room temperature and then refluxed for 3 hours. The mixture was diluted with water, alkalinized with 10 N sodium hydroxide solution and extracted three times with methylene chloride. The combined organic phase was washed with water, dried ($Na_2SO_4$) and evaporated giving 2.8 g of crude product which was recrystallized from light petroleum-/ethyl acetate (5/1). Yield 2.1 g (72%). M.p. 164°C.

v) cis-4b,5,9b,10-tetrahydro-8-ethoxy-4b,7,9,9b-tetramethylindeno[1,2-b]indole.

To a cold (-78°C) solution of 2.0 g (0.0063 mol) of 9b,10-dihydro-8-ethoxy-7,9,9b-trimethylindeno[1,2-b]indole in 20 ml of dry tetrahydrofuran, 10 ml of methyllithium (1.6 N) in ether was added dropwise with stirring and under argon. After the addition was complete the stirring was continued for 1 hour at -20°C and then for 1 hour at room temperature. The reaction mixture was quenched by addition of 20 ml of saturated ammonium chloride solution. After addition of 100 ml of ether the organic phase was separated and washed twice with ammonium chloride solution. Drying ($Na_2SO_4$) and evaporation gave 2.0 g (96%) of the product. 1H NMR ($CDCl_3$): 1.35 (3H,t), 1.4 (6H,d), 2.1 (3H,s), 2.35 (3H,s), 2.95-3.05 (1H,d), 3.55-3.75 (3H,m), 6.1 (1H,s), 7.0-7.3 (4H,m).

## EXAMPLE 3

Cis-4b,5,9b,10-tetrahydro-8-methoxy-6,7,9-trimethylindeno[1,2-b]indole.

i) 5,10-dihydro-8-methoxy-6,7,9-trimethylindeno-[1,2-b]indole.

A mixture of 1.95 g (0.009 mol) of 4-hydrazino-2,3,6-trimethylanisole hydrochloride (prepared as described in Example 1) and 1.06 g (0.008 mol) of 1-indanone in 20 ml of ethanol and 2 ml of concentrated hydrochloric acid was refluxed for 1 hour. The solvent was removed by evaporation and the residue partitioned between ether and water. The aqueous phase was made alkaline with sodium hydroxide solution. The organic phase was separated and washed with water. Drying ($Na_2SO_4$) and evaporation gave the crude product which was purified by chromatography using methylene chloride/light petroleum (20/80) as eluant. A final recrystallization gave 1.08 g (49%) of the product.

ii) cis-4b,5,9b,10-tetrahydro-8-methoxy-6,7,9-trimethylindeno[1,2-b]indole.

To a solution of 0.65 g (0.00234 mol) of 5,10-dihydro-8-methoxy-6,7,9-trimethylindeno[1,2-b]indole and 0.95 g (0.00957 mol) of morpholino borane in 4 ml of dioxane was added dropwise 1 ml of concentrated hydrochloric acid. The mixture was refluxed for 30 minutes, cooled to room temperature whereupon 3 ml of 6 N hydrochloric acid was added. The mixture was then refluxed for another 30 minutes. After cooling to room temperature, the crude mixture was partitioned between ether and aqueous sodium hydroxide. The organic phase was separated and washed with aqueous sodium hydroxide and with water. Drying ($MgSO_4$) and evaporation gave the crude product which was recrystallized from ethyl acetate/light petroleum. Yield 0.46 g (70%). $^1H$ NMR ($CDCl_3$): 2.0 (3H,s), 2.14 (3H,s), 2.28 (3H,s), 3.17 (1H,dd), 3.55 (1H,dd), 3.64 (1H,s), 4.28 (1H,ddd), 5.38 (1H,d), 7.20-7.27 (3H,m), 7.39 (1H,d).

## Pharmacological Properties

The indenoindoles according to in the present invention are hydrophobic and stable structures which form cations, stable cation radicals or radicals upon oxidation. They constitute potent antioxidants as measured by inhibition of $Fe^{2+}$-ascorbate induced lipid peroxidation in vitro, with $IC_{50}$ value as low as 10 nM. The compounds of formula I prevent efficiently oxidation of lipoproteins in human plasma in the presence of rabbit smooth muscle cells or mouse peritoneal macrophages. They also prevent ischemic/reperfusion damage to the isolated perfused rat heart, and protect against carbon tetrachloride-, acetaminophen-, methylmethane sulfonate-, menadione-, t-butyl hydroperoxide-, and N-methyl-$N^1$-nitro-N-nitro-soguanidine-induced liver damage in mice, or in

isolated rat hepatocytes.

These properties suggest that the compounds of the invention have a potential use in the protection or treatment of ischemic or reperfusion injury, particularly cerebral and cardiac ischemia/infarct, atherosclerosis, thrombosis, embolism, Parkinson's disease, ageing, Alzheimer's disease, neoplasms and toxicity of anti-neoplastic drugs, immunosuppresive agents and inflammation including allergic/inflammatory conditions like bronchial asthma and rheumatoid arthritis. Other potential applications are chemoprevention against chemical toxicity or radiation damage. The indenoindole compounds are not appreciably activated by UV light making them candidates for use in skin care products. Another interesting and important feature of the indenoindole compounds of the present invention is their ability to stabilize membranes.

Pharmacological Tests

The most remarkable feature of the compounds of the invention is their efficacy as free-radical scavengers or antioxidants. An assay system measuring the concentration of the compounds of formula I required to inhibit lipid peroxidation by 50% ($IC_{50}$) was used. The lipid peroxidation assay is described below under 1. and the data presented in Table 1. Additionally a test of inhibition of macrophage induced LDL-peroxidation is described below under 2. and the data is presented in Table 2.

1. Ascorbate/$Fe^{2+}$-dependent lipid peroxidation

For the ferrous/ascorbate lipid peroxidation system, 6.25 ml of 0.1 M potassium phosphate buffer ($KP_i$), pH 7.4, was added to 12.5 mg dried soy bean phospholipids. After flushing with argon for 2 min, the suspension was sealed with five layers of Parafilm and sonicated until the suspension was translucent. The final reaction mixture was composed of 200 μg/ml phospholipid, 10 μM $FeNH_4(SO_4)_2$ or $Fe(NH_4)_2(SO_4)_2$, and 100 μM ascorbic acid in 0.1 M $KP_i$ (pH 7.4), and the antioxidant to be tested in acetone or DMSO. The volume of vehicle never exceeded 1% of the total volume. The reaction was initiated by the addition of ascorbic acid plus iron. The reaction was continued at room temperature in a shaking water bath for 30 min and then stopped by the addition of 10 μM of 0.5 M butylated hydroxytoluene in DMSO. The above procedure and the subsequent determination of 2-thiobarbituric acid-reactive material is described in: Shertzer, H.G. et al, Biochem. Pharmacol. 37, 333 (1988). Table 1 shows the effects of indenoindoles and α-tocopherol on ascorbate/$Fe^{2+}$-dependent lipid peroxidation.

Table 1

| Compounds | $pIC_{50}$ |
|---|---|
| Cis-8-methoxy-6,7,9-trimethyl-THII | 8.0 |
| Cis-8-ethoxy-4b,7,9,9b-tetramethyl-THII | 7.9 |
| Cis-8-methoxy-4b,6,7,9,9b-pentamethyl-THII | 7.6 |

2. Inhibition of macrophage induced LDL-peroxidation.

Mouse peritonal macrophages were incubated in Ham's F10 medium in the presence of 25 μg of human low density lipoproteins (LDL)/ml of macrophage medium. The compounds of the invention were dissolved in ethanol and added to a final concentration of $10^{-5}$ to $10^{-10}$ M. The cells in triplicate were then incubated for 24 hours. The cell medium was then removed and the lipid peroxidation was assayed by measuring the formation of thio-barbituric acid reactive substances (TBARS) using a method described by Steinbrecher et al in Proc. Natl. Acad. Sci. USA 81, 3883 (1984). The results are given in Table 2 as the -log of the concentrations necessary to reduce lipid peroxidation by 50% compared to control ($pIC_{50}$).

Table 2

| Compounds | $pIC_{50}$ |
|---|---|
| Cis-8-methoxy-4b,6,7,9,9b-pentamethyl-THII | 7.9 |
| Cis-8-ethoxy-4b,7,9,9b-tetramethyl-THII | 7.8 |
| Cis-8-methoxy-6,7,9-trimethyl-THII | 7.9 |

## Claims

1. A cis-fused compound of the formula I

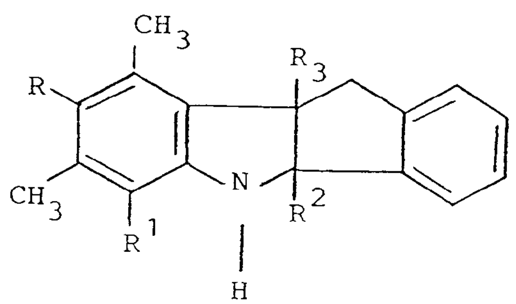

I

wherein R is methoxy or ethoxy, when R is methoxy, $R^1$ is methyl and $R^2$ and $R^3$ are both hydrogen or both methyl, and when R is ethoxy, $R^1$ is hydrogen and $R^2$ and $R^3$ are both methyl, or an enantiomer or salt thereof.

2. Cis-4b,5,9b,10-tetrahydro-8-ethoxy-4b,7,9,9b-tetramethylindeno[1,2-b]indole, enantiomers and salts thereof.

3. Cis-4b,5,9b,10-tetrahydro-8-methoxy-6,7,9-trimethylindeno[1,2-b]indole, enantiomers and salts thereof.

4. Cis-4b,5,9b,10-tetrahydro-8-methoxy-4b,6,7,9,9b-pentamethylindeno[1,2-b]indole, enantiomers and salts thereof.

5. A compound as defined in any preceding claim, an enantiomer or pharmaceutically acceptable salt thereof for use in a method of therapy practised on the human or animal body.

6. A compound according to claim 5 for use in the treatment of atherosclerosis.

7. A compound according to claim 5 for use in the treatment of ischemic or reperfusion injuries, thrombosis, and embolism.

8. A compound according to claim 5 for use in the treatment or prevention of neoplasms, or for use in the treatment of Parkinson's disease, Alzheimer's disease or ageing, of allergic/inflammatory conditions such as bronchial asthma and rheumatoid arthritis, or of damages caused by chemicals, radiation, antineoplastic or immunosuppressive agents.

9. A pharmaceutical composition comprising an active ingredient which is a compound as defined in any one of claims 1 to 4, an enantiomer or a pharmaceutically acceptable salt thereof.

10. Use of a compound as defined in any one of claims 1 to 4, an enantiomer or a pharmaceutically acceptable salt thereof in the manufacture of a medicament for treatment of ischemic or reperfusion injuries, thrombosis, embolism, atherosclerosis, Parkinson's disease, Alzheimer's disease, ageing, neoplasms, allergic or inflammatory conditions such as bronchial asthma, and rheumatoid arthritis, and damage caused by chemicals, radiation, antineoplastic or immunosuppressive agents.

11. A process for preparation of a compound as defined in any one of claims 1 to 4 by:
a. reduction of the 5,10-dihydroindeno[1,2-b]indole (DHII)

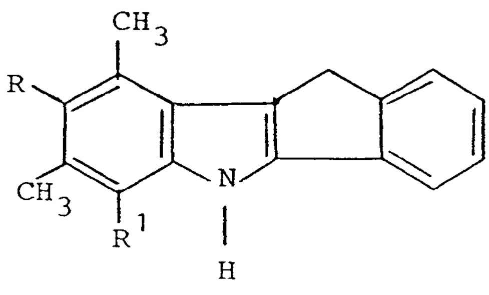

II

wherein R and $R^1$ are as defined in claim 1,
b. reaction of an indolenine of formula III with methyl lithium ($CH_3Li$)

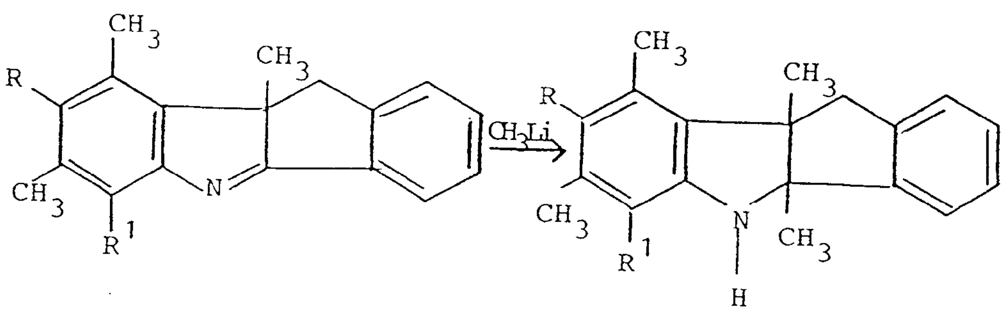


III IB

wherein R and $R^1$ are as defined in formula I,
c. for 4b,9b-dimethyl compounds, by a sequence of metallation of the corresponding 4b-unsubstituted analogue of the formula VII

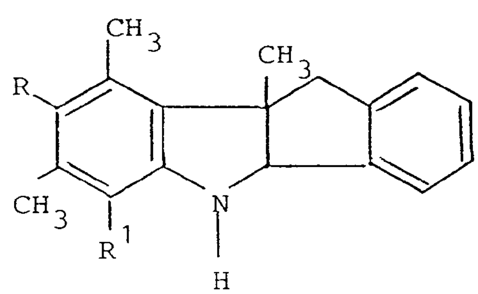

VII

followed by carbonation with carbon dioxide, metallation and alkylation with a methyl-halide or methyl-sulphate and a final hydrolysis.

12. A composition comprising a compound susceptible to oxidative deterioration and a compound as defined in any one of claims 1 to 4 or an enantiomer or salt thereof.

13. A method of stabilising a compound susceptible to oxidative deterioration by contacting the susceptible compound with a compound as defined in any one of claims 1 to 4 or an enantiomer or salt thereof.

European Patent Office

# EUROPEAN SEARCH REPORT

Application Number

EP 91 30 9445

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | FASEB JOURNAL.<br>1988, BETHESDA, MD US<br>page A407;<br>H.G. SHERTZER ET AL.: 'Amelioration of carbon tetrachloride hepatotoxicity in mice by indenoindole compounds'<br>* 648 *<br>--- | 1,10 | C07D209/94<br>A61K31/40 |
| A | CHEMICAL ABSTRACTS, vol. 109,<br>1988, Columbus, Ohio, US;<br>abstract no. 224224H,<br>SHERTZER,H.G.: 'protection against carbon tetrachloride hepatotoxicity by 5,10-dihydroindeno(1,2-b)indole, a potent inhibitor of lipid peroxidation.'<br>page 248 ;<br>* abstract *<br>----- | 1,10 | |
| | | | TECHNICAL FIELDS SEARCHED (Int. Cl.5 ) |
| | | | C07D |

The present search report has been drawn up for all claims

| Place of search | Date of completion of the search | Examiner |
|---|---|---|
| THE HAGUE | 03 JANUARY 1992 | VAN BIJLEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)